# EUROPEAN PATENT APPLICATION

(11) **EP 4 316 291 A1**
(43) Date of publication of application: **07.02.2024**
(21) Application number: 22911580.3
(22) Date of filing: 28.10.2022
(51) Int. Cl.: A24F 40/60, A24F 40/65, A24F 40/51, A24F 40/50, A24F 40/53, H04W 88/02, G08B 5/22

(54) **METHOD AND DEVICE FOR OUTPUTTING INHALATION ABILITY STATE**

(30) Priority: 23.12.2021 KR 20210186170
(71) Applicant: KT & G Corporation, Daejeon 34337 (KR)
(72) Inventor: KI, Sung Jong, Daejeon 34128 (KR); LEE, Sang Hyun, Daejeon 34128 (KR); JEONG, Jaeseong, Daejeon 34128 (KR)
(74) Representative: Ter Meer Steinmeister & Partner
(86) International application number: PCT/KR2022/016662
(87) International publication number: WO 2023/120942

(57) **Abstract**

According to an embodiment, in order to output a status of a suction ability of a user, inhalation information generated based on one or more sensors of an additional device may be received from the additional device connected to an electronic device, the suction ability of the user may be determined based on the inhalation information, a status of the user may be determined by evaluating the suction ability based on a reference suction ability, and a notification message may be generated and output based on the status.

## Description

### Technical Field

The following embodiments relate to a technique for outputting a status of a suction ability of a user, and more particularly, to a technique for determining the status of the suction ability based on inhalation information of an electronic device.

### Background Art

These days, there is a gradual rise in the demand for electronic cigarettes. The rising demand for electronic cigarettes has accelerated the continued development of electronic cigarette related functions. The electronic cigarette-related functions may include, in particular, functions according to types and characteristics of electronic cigarettes.

### Disclosure of the Invention

### Technical Goals

An embodiment may provide a method of outputting a status of a suction ability performed by an electronic device.

An embodiment may provide an electronic device that outputs a status of a suction ability.

### Technical Solutions

According to an embodiment, a method of outputting a status of a suction ability, performed by an electronic device may include receiving, from an additional device connected to the electronic device, inhalation information generated based on one or more sensors in the additional device, determining a target suction ability of a user based on the inhalation information, determining a status of the user by evaluating the target suction ability based on a reference suction ability preset in the electronic device, generating a notification message based on the status, and outputting the notification message.

The inhalation information may include at least one of an average flow rate of airflow according to inhalation by the user, a cross-sectional area of a channel through which airflow passes, and a duration of resistance to draw.

The method of outputting the status of the suction ability may further include calculating the reference suction ability based on lung capacity information of the user received from the user or an external device.

The determining of the status of the user by evaluating the target suction ability based on the reference suction ability preset in the electronic device may include determining the status of the user by comparing a reference suction intensity and a reference suction time of the reference suction ability with a target suction intensity and a target suction time of the target suction ability.

The notification message may indicate a message to adjust at least one of a suction intensity and a suction time.

The method of outputting the status of the suction ability may include storing the target suction ability and providing the user with a history of a suction ability of the user based on the target suction ability.

The electronic device may be a mobile communication terminal, and the additional device may be an electronic cigarette.

According to an embodiment, an electronic device may include a memory configured to store a program for outputting a status of a suction ability and a processor configured to perform the program, wherein the processor may be configured to receive, from an additional device connected to the electronic device, inhalation information generated based on one or more sensors of the additional device, determine a target suction ability of a user based on the inhalation information, determine a status of the user by evaluating the target suction ability based on a reference suction ability preset in the electronic device, generate a notification message based on the status, and output the notification message.

The electronic device may be a mobile communication terminal, and the additional device may be an electronic cigarette.

### Effects

A method of outputting a status of a suction ability performed by an electronic device may be provided.

An electronic device for outputting a status of a suction ability may be provided.

### Brief Description of Drawings

FIG. 1 illustrates a system for managing smoking information according to an embodiment.
FIGS. 2 to 4 are diagrams illustrating examples of a cigarette inserted into an aerosol generating device according to an example.
FIGS. 5 and 6 are views of examples of a cigarette according to an example.
FIG. 7 is a block diagram illustrating an aerosol generating device according to another example.
FIG. 8 is a block diagram illustrating an aerosol generating device according to still another example.
FIG. 9 is a diagram illustrating a configuration of a user terminal according to an embodiment.
FIG. 10 is a flowchart illustrating a method of outputting a status of a suction ability performed by a user terminal according to an embodiment.
FIG. 11 is a flowchart illustrating a method of calculating a reference suction ability according to an example.
FIG. 12 is a flowchart illustrating a method of providing a history of a suction ability of a user according to an example.

### Best Mode for Carrying Out the Invention

The following detailed structural or functional description is provided as an example only and various alterations and modifications may be made to embodiments. Accordingly, the embodiments are not to be construed as limited to the disclosure and should be understood to include all changes, equivalents, or replacements within the idea and the technical scope of the disclosure.

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that, if one component is described as being "connected," "coupled," or "joined" to another component, a third component may be "connected," "coupled," and "joined" between the first and second components, although the first component may be directly connected, coupled, or joined to the second component.

The singular forms "a," "an," and "the" are intended to include the plural forms as well, unless the context clearly indicates otherwise. As used herein, the terms "include," "comprise," and "have" specify the presence of stated features, numbers, operations, elements, components, or combinations thereof, but do not preclude the presence or addition of one or more other features, numbers, operations, elements, components, or combinations thereof.

Unless otherwise defined, all terms, including technical and scientific terms, used herein have the same meaning as commonly understood by one of ordinary skill in the art to which the present disclosure pertains. Terms, such as those defined in commonly used dictionaries, are to be construed to have meanings matching with contextual meanings in the relevant art, and are not to be construed to have an ideal or excessively formal meaning unless otherwise defined herein.

Hereinafter, embodiments will be described in detail with reference to the accompanying drawings. When describing the embodiments with reference to the accompanying drawings, like reference numerals refer to like elements and a repeated description related thereto will be omitted.

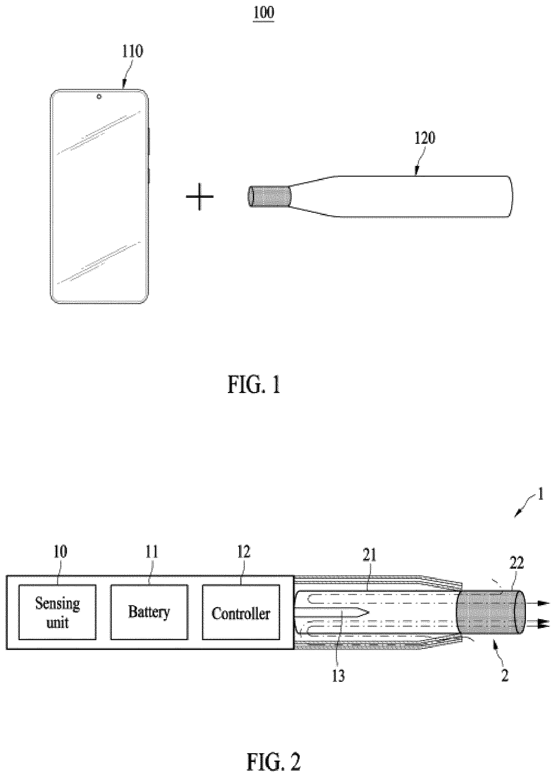

FIG. 1 illustrates a system for managing smoking information according to an embodiment. According to an embodiment, a system 100 for outputting a suction ability of a user may include a user terminal 110 and an aerosol generating device 120. Each of the user terminal 110 and the aerosol generating device 120 may be an electronic device.

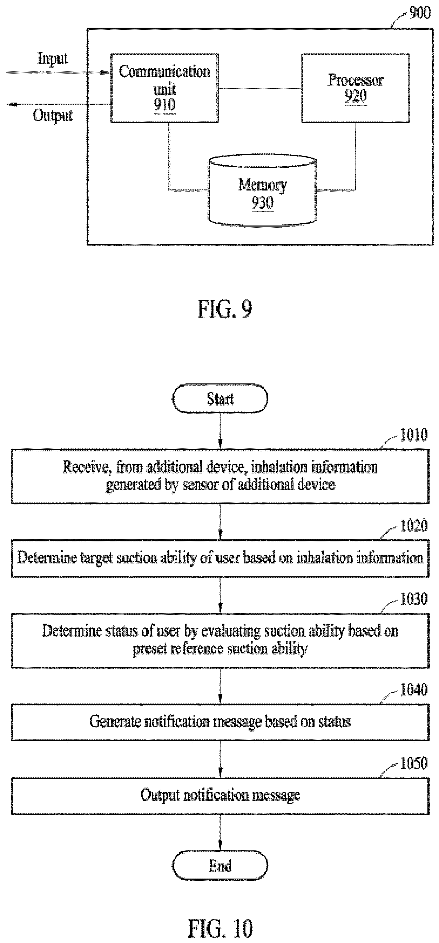

For example, the user terminal 110 may be a mobile communication terminal. The configuration of the user terminal 110 will be described in detail with reference to FIG. 9.

For example, the aerosol generating device 120 may be referred to as an electronic cigarette device or a smoking stick. The aerosol generating device 120 will be described in detail below with reference to FIGS. 2 to 8.

The user may smoke as being provided with an aerosol generated by the aerosol generating device 120. For example, the aerosol generating device 120 may generate an aerosol by heating a cigarette inserted into the aerosol generating device 120. As another example, the aerosol generating device 120 may generate an aerosol using a material in a liquid-type cartridge or a replace solid-type cartridge in the aerosol generating device 120. The method by which the aerosol generating device 120 generates an aerosol is not limited to the embodiments described above.

According to an embodiment, the aerosol generating device 120 may generate sensing information related to a status of the aerosol generating device 120 using various sensors included in the aerosol generating device 120, and transmit the sensing information to the user terminal 110. For example, the sensing information may include inhalation information of the user using the aerosol generating device 120. The user terminal 110 may extract health data related to the lung capacity of the user or air intake through the inhalation information, and based thereon, provide a health care-related service to the user. A method of outputting a status of the suction ability of the user will be described in detail below with reference to FIGS. 10 to 12. FIGS. 2 to 4 are diagrams illustrating examples of a cigarette inserted into an aerosol generating device according to an example.

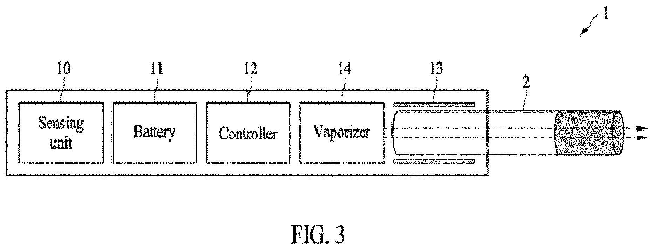

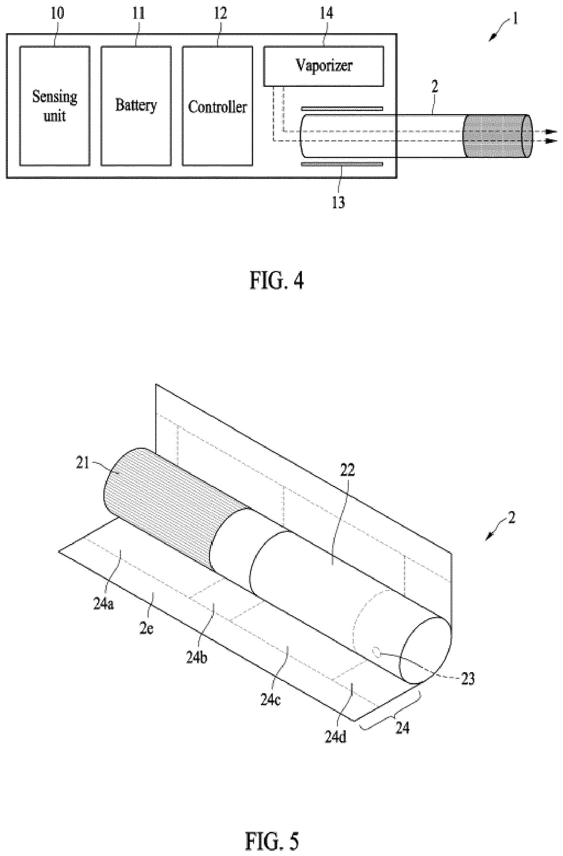

Referring to FIG. 2, an aerosol generating device 1 may include a sensing unit 10, a battery 11, a controller 12, and a heater 13. Referring to FIGS. 3 and 4, the aerosol generating device 1 may further include a vaporizer 14. Further, a cigarette 2 may be inserted into an inner space of the aerosol generating device 1. The aerosol generating device 1 may be the aerosol generating device 120 described above with reference to FIG. 1.

According to an embodiment, the aerosol generating device 1 may further include a display.

The aerosol generating device 1 shown in FIGS. 2 to 4 may include components related to the embodiments described herein. Therefore, it is to be understood by one of ordinary skill in the art to which the present disclosure pertains that the aerosol generating device 1 may further include other generally used components in addition to the ones shown in FIGS. 2 to 4.

In addition, although it is shown that the heater 13 is included in the aerosol generating device 1 in FIGS. 3 and 4, the heater 13 may be omitted as needed.

FIG. 2 illustrates a linear alignment of the sensing unit 10, the battery 11, the controller 12, and the heater 13. In addition, FIG. 3 illustrates a linear alignment of the battery 11, the controller 12, the vaporizer 14, and the heater 13. Further, FIG. 4 illustrates a parallel alignment of the vaporizer 14 and the heater 13. However, the internal structure of the aerosol generating device 1 is not limited to what is shown in FIGS. 2 to 4. That is, such alignments of the sensing unit 10, the battery 11, the controller 12, the heater 13, and the vaporizer 14 may be changed depending on the design of the aerosol generating device 1.

When the cigarette 2 is inserted into the aerosol generating device 1, the aerosol generating device 1 may actuate the heater 13 and/or the vaporizer 14 to generate an aerosol. The aerosol generated by the heater 13 and/or the vaporizer 14 may pass through the cigarette 2 into the user. Even when the cigarette 2 is not inserted in the aerosol generating device 1, the aerosol generating device 1 may heat the heater 13, as needed.

The battery 11 may supply power to be used to operate the aerosol generating device 1. For example, the battery 11 may supply power to heat the heater 13 or the vaporizer 14, and may supply power required for the controller 12 to operate. In addition, the battery 11 may supply power required to operate a display, a sensor, a motor, or the like installed in the aerosol generating device 1.

The controller 12 may control the overall operation of the aerosol generating device 1. For example, the controller 12 may control respective operations of other components included in the aerosol generating device 1, in addition to the sensing unit 10, the battery 11, the heater 13, and the vaporizer 14. In addition, the controller 12 may verify a state of each of the components of the aerosol generating device 1 to determine whether the aerosol generating device 1 is in an operable state.

The controller 12 may include at least one processor. The processor may be implemented as an array of a plurality of logic gates, or may be implemented as a combination of a general-purpose microprocessor and a memory in which a program executable in the microprocessor is stored. In addition, it is to be understood by one of ordinary skill in the art to which the present disclosure pertains that the processor may be implemented in other types of hardware.

The heater 13 may be heated by power supplied by the battery 11. For example, when the cigarette is inserted into the aerosol generating device 1, the heater 13 may be disposed outside the cigarette. The heated heater 13 may thus raise the temperature of an aerosol generating material in the cigarette.

The heater 13 may be an electrically resistive heater. For example, the heater 13 may include an electrically conductive track, and the heater 13 may be heated as a current flows through the electrically conductive track. However, the heater 13 is not limited to the foregoing example, and any example of heating the heater 13 up to a desired temperature may be applicable without limitation. The desired temperature may be preset in the aerosol generating device 1 or may be set by the user.

As another example, the heater 13 may be an induction heater. Specifically, the heater 13 may include an electrically conductive coil for heating the cigarette in an inductive heating manner, and the cigarette may include a susceptor to be heated by the induction heater.

For example, the heater 13 may include a tubular heating element, a plate-shaped heating element, a needle-shaped heating element, or a rod-shaped heating element, and may heat the inside or outside of the cigarette 2 according to the shape of a heating element.

In addition, the heater 13 may be provided as a plurality of heaters in the aerosol generating device 1. In this case, the plurality of heaters 13 may be disposed to be inserted into the cigarette 2, or may be disposed outside the cigarette 2. In addition, some of the heaters 13 may be disposed to be inserted into the cigarette 2, and the rest may be disposed outside the cigarette 2. However, the shape of the heater 13 is not limited to what is shown in FIGS. 2 to 4 but may be provided in various shapes.

The vaporizer 14 may heat a liquid composition to generate an aerosol, and the generated aerosol may pass through the cigarette 2 into the user. That is, the aerosol generated by the vaporizer 14 may travel along an airflow path of the aerosol generating device 1, and the airflow path may be configured such that the aerosol generated by the vaporizer 14 passes through the cigarette into the user.

For example, the vaporizer 14 may include a liquid storage, a liquid transfer means, and a heating element, but is not limited thereto. For example, the liquid storage, the liquid transfer means, and the heating element may be included as independent modules in the aerosol generating device 1.

The liquid storage may store the liquid composition. The liquid composition may be, for example, a liquid including a tobacco-containing material that includes a volatile tobacco flavor component, or may be a liquid including a non-tobacco material. The liquid storage may be manufactured to be detachable and attachable from and to the vaporizer 14, or may be manufactured in an integral form with the vaporizer 14.

The liquid composition may include, for example, water, a solvent, ethanol, a plant extract, a fragrance, a flavoring agent, or a vitamin mixture. The fragrance may include, for example, menthol, peppermint, spearmint oil, various fruit-flavored ingredients, and the like. However, embodiments are not limited thereto. The flavoring agent may include ingredients that provide a user with a variety of flavors or scents. The vitamin mixture may be a mixture of at least one of vitamin A, vitamin B, vitamin C, and vitamin E. However, embodiments are not limited thereto. The liquid composition may also include an aerosol former such as glycerin and propylene glycol.

The liquid transfer means may transfer the liquid composition in the liquid storage to the heating element. The liquid transfer means may be, for example, a wick such as cotton fiber, ceramic fiber, glass fiber, or porous ceramic. However, embodiments are not limited thereto.

The heating element may be an element for heating the liquid composition transferred by the liquid transfer means. The heating element may be, for example, a metal heating wire, a metal heating plate, a ceramic heater, or the like. However, embodiments are not limited thereto. In addition, the heating element may include a conductive filament such as a nichrome wire, and may be arranged in a structure wound around the liquid transfer means. The heating element may be heated as a current is supplied and may transfer heat to the liquid composition in contact with the heating element, and may thereby heat the liquid composition. As a result, an aerosol may be generated.

For example, the vaporizer 14 may be referred to as a cartomizer or an atomizer. However, embodiments are not limited thereto.

Meanwhile, the aerosol generating device 1 may further include general-purpose components in addition to the sensing unit 10, the battery 11, the controller 12, the heater 13, and the vaporizer 14. For example, the aerosol generating device 1 may include a display that outputs visual information and/or a motor that outputs tactile information.

According to an embodiment, the sensing unit 10 may include one or more biosensors. For example, the biosensors may include one or more of a blood pressure sensor, an electrocardiogram sensor, or a blood oxygen saturation sensor. The biosensors may include sensors configured to measure biosignals of the user, and are not limited to the embodiments described above. When the user grabs the aerosol generating device 1, a biosignal of the user may be measured.

According to an embodiment, the sensing unit 10 may include a sensor configured to measure a body composition of the user. For example, the body composition may include a skeletal muscle mass, a basal metabolic rate, a body water content, and a body fat mass. When the user grabs the aerosol generating device 1, the body composition of the user may be measured.

According to an embodiment, the sensing unit 10 may further include a puff detection sensor, a temperature detection sensor, a cigarette insertion detection sensor. In addition, the aerosol generating device 1 may be manufactured to have a structure in which external air may be introduced or internal gas may flow out even with the cigarette 2 being inserted.

Although not shown in FIGS. 2 to 4, the aerosol generating device 1 may constitute a system along with a separate cradle. For example, the cradle may be used to charge the battery 11 of the aerosol generating device 1. Alternatively, when the cradle and the aerosol generating device 1 are coupled to each other, the heater 13 may be heated.

The cigarette 2 may be similar to a general combustible cigarette. For example, the cigarette 2 may be divided into a first portion including an aerosol generating material and a second portion including a filter or the like. Alternatively, the second portion of the cigarette 2 may also include the aerosol generating material. For example, the aerosol generating material provided in the form of granules or capsules may be inserted into the second portion.

The first portion may be entirely inserted into the aerosol generating device 1, and the second portion may be exposed outside. Alternatively, the first portion may be partially inserted into the aerosol generating device 1, and the first portion may be entirely inserted, and the second portion may be partially inserted into the aerosol generating device 1. The user may then suck an aerosol with the second portion in a mouth of the user. In this case, an aerosol may be generated as external air passes through the first portion, and the generated aerosol may pass through the second portion into the mouth of the user.

For example, the external air may be introduced through at least one air path formed in the aerosol generating device 1. In this example, the opening or closing and/or the size of the air path formed in the aerosol generating device 1 may be adjusted by the user. Accordingly, an amount of atomization, a sense of smoking, or the like may be adjusted by the user. As another example, the external air may be introduced into the inside of the cigarette 2 through at least one hole formed on a surface of the cigarette 2.

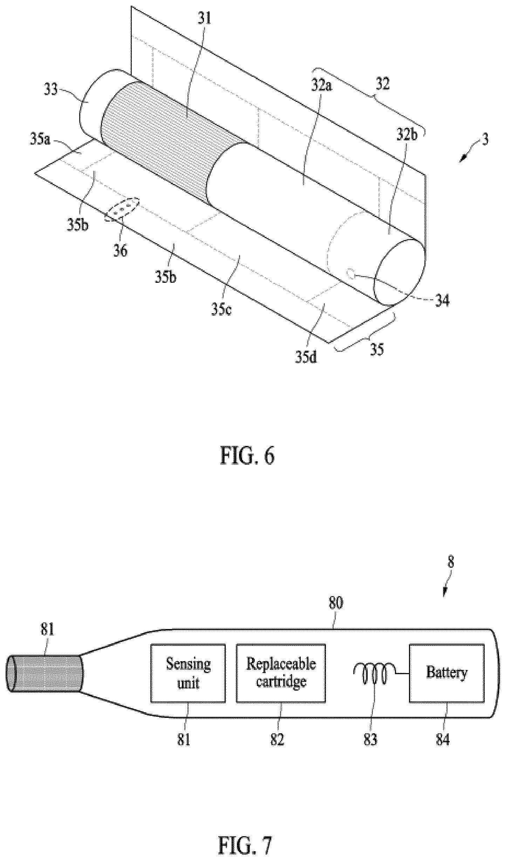

Hereinafter, examples of the cigarette 2 will be described with reference to FIGS. 5 and 6.

FIGS. 5 and 6 are views of examples of a cigarette according to an example.

Referring to FIG. 5, the cigarette 2 may include a tobacco rod 21 and a filter rod 22. The first portion and the second portion described above with reference to FIGS. 2 to 4 may include the tobacco rod 21 and the filter rod 22, respectively.

Although the filter rod 22 is illustrated as having a single segment in FIG. 5, embodiments are not limited thereto. That is, the filter rod 22 may include a plurality of segments. For example, the filter rod 22 may include a segment that cools an aerosol and a segment that filters a predetermined ingredient contained in an aerosol. In addition, the filter rod 22 may further include at least one segment that performs another function as needed.

A diameter of the cigarette 2 may be in a range of 5 millimeters (mm) to 9 mm, and a length thereof may be about 48 mm. However, embodiments are not limited thereto. For example, a length of the tobacco rod 21 may be about 12 mm, a length of a first segment of the filter rod 22 may be about 10 mm, a length of a second segment of the filter rod 22 may be about 14 mm, and a length of a third segment of the filter rod 22 may be about 12 mm. However, embodiments are not limited thereto.

The cigarette 2 may be wrapped with at least one wrapper 24. The wrapper 24 may have at least one hole through which external air is introduced or internal gas flows out. For example, the cigarette 2 may be wrapped with one wrapper 24. As another example, the cigarette 2 may be wrapped with two or more wrappers 24 in an overlapping manner. For example, the tobacco rod 21 may be wrapped with a first wrapper 24a, and the filter rod 22 may be wrapped with wrappers 24b, 24c, and 24d. In addition, the cigarette 2 may be entirely wrapped again with a single wrapper 24e. For example, when the filter rod 22 includes a plurality of segments, the segments may be wrapped with the wrappers 24b, 24c, and 24d, respectively.

The first wrapper 24a and the second wrapper 24b may be formed of general filter wrapping paper. For example, the first wrapper 24a and the second wrapper 24b may be porous wrapping paper or non-porous wrapping paper. In addition, the first wrapper 24a and the second wrapper 24b may be formed of oilproof paper and/or an aluminum laminated wrapping material.

The third wrapper 24c may be formed of hard wrapping paper. For example, a basis weight of the third wrapper 24c may be in a range of 88 grams per square meter (g/m²) to 96 g/m², and desirably, may be in a range of 90 g/m² to 94 g/m². In addition, a thickness of the third wrapper 24c may be in a range of 120 micrometers (µm) to 130 µm, and desirably, may be 125 µm.

The fourth wrapper 24d may be formed of oilproof hard wrapping paper. For example, a basis weight of the fourth wrapper 24d may be in a range of 88 g/m² to 96 g/m², and desirably, may be in a range of 90 g/m² to 94 g/m². In addition, a thickness of the fourth wrapper 24d may be in a range of 120 µm to 130 µm, and desirably, may be 125 µm.

The fifth wrapper 24e may be formed of sterile paper (e.g., MFW). Here, the sterile paper (MFW) may refer to paper specially prepared to enhance tensile strength, water resistance, smoothness, or the like, compared to general paper. For example, a basis weight of the fifth wrapper 24e may be in a range of 57 g/m² to 63 g/m², and desirably, may be 60 g/m². In addition, a thickness of the fifth wrapper 24e may be in a range of 64 µm to 70 µm, and desirably, may be 67 µm.

The fifth wrapper 24e may have a predetermined material internally added thereto. The predetermined material may be, for example, silicon. However, embodiments are not limited thereto. Silicon may have properties, such as, for example, heat resistance which is characterized by less change by temperature, oxidation resistance which refers to resistance to oxidation, resistance to various chemicals, water repellency against water, or electrical insulation. However, silicon may not be necessarily used, but any material having such properties described above may be applied (or coated) to the fifth wrapper 24e without limitation.

The fifth wrapper 24e may prevent the cigarette 2 from burning. For example, there may be a probability that the cigarette 2 burns when the tobacco rod 21 is heated by the heater 13. For example, when the temperature rises above an ignition point of any one of materials included in the tobacco rod 21, the cigarette 2 may burn. Even in this case, it may still be possible to prevent the cigarette 2 from burning because the fifth wrapper 24e includes a non-combustible material. In addition, the fifth wrapper 24e may prevent a holder from being contaminated by substances produced in the cigarette 2. For example, liquid substances may be produced in the cigarette 2 when a user puffs. For example, as an aerosol generated in the cigarette 2 is cooled by external air, such liquid substances (e.g., water, etc.) may be produced. Thus, wrapping the cigarette 2 with the fifth wrapper 24e may prevent the liquid substances produced in the cigarette 2 from leaking out of the cigarette 2.

The tobacco rod 21 may include an aerosol generating material. The aerosol generating material may include, for example, at least one of glycerin, propylene glycol, ethylene glycol, dipropylene glycol, diethylene glycol, triethylene glycol, tetraethylene glycol, or oleyl alcohol. However, embodiments are not limited thereto. The tobacco rod 21 may also include other additives, such as, for example, a flavoring agent, a wetting agent, and/or an organic acid. In addition, the tobacco rod 21 may include a flavoring liquid such as menthol or a moisturizing agent that is added as being sprayed onto the tobacco rod 21.

The tobacco rod 21 may be manufactured in various forms. For example, the tobacco rod 21 may be manufactured as a sheet or as a strand. The tobacco rod 21 may also be formed of tobacco leaves finely cut from a tobacco sheet. In addition, the tobacco rod 21 may be enveloped by a thermally conductive material. The thermally conductive material may be, for example, metal foil such as aluminum foil. However, embodiments are not limited thereto. For example, the thermally conductive material enveloping the tobacco rod 21 may evenly distribute the heat transferred to the tobacco rod 21 to improve the thermal conductivity to be applied to the tobacco rod, thereby improving the taste of tobacco. In addition, the thermally conductive material enveloping the tobacco rod 21 may function as a susceptor heated by an induction heater. In this case, although not shown, the tobacco rod 21 may further include an additional susceptor in addition to the thermally conductive material enveloping the outside thereof.

The filter rod 22 may be a cellulose acetate filter. However, a shape of the filter rod 22 is not limited. For example, the filter rod 22 may be a cylindrical rod or a tubular rod including a hollow therein. The filter rod 22 may also be a recess-type rod. For example, when the filter rod 22 includes a plurality of segments, at least one of the segments may be manufactured in a different shape.

The first segment of the filter rod 22 may be a cellulose acetate filter. For example, the first segment may be a tubular structure including a hollow therein. The first segment may prevent internal materials of the tobacco rod 21 from being pushed back when the heater 13 is inserted into the tobacco rod 21 and may cool the aerosol. A desirable diameter of the hollow included in the first segment may be adopted from a range of 2 mm to 4.5 mm. However, embodiments are not limited thereto.

A desirable length of the first segment may be adopted from a range of 4 mm to 30 mm. However, embodiments are not limited thereto. Desirably, the length of the first segment may be 10 mm. However, embodiments are not limited thereto.

The first segment may have a hardness that is adjustable through an adjustment of the content of a plasticizer in a process of manufacturing the first segment. In addition, the first segment may be manufactured by inserting a structure such as a film or a tube of the same or different materials therein (e.g., in the hollow).

A second segment of the filter rod 22 may cool an aerosol generated as the heater 13 heats the tobacco rod 21. The user may thus suck the aerosol cooled down to a suitable temperature.

A length or diameter of the second segment may be determined in various ways according to the shape of the cigarette 2. For example, a desirable length of the second segment may be adopted from a range of 7 mm to 20 mm. Desirably, the length of the second segment may be about 14 mm. However, embodiments are not limited thereto.

The second segment may be manufactured by weaving a polymer fiber. In this case, a flavoring liquid may be applied to a fiber formed of a polymer. Alternatively, the second segment may be manufactured by weaving a separate fiber to which a flavoring liquid is applied and the fiber formed of the polymer together. Alternatively, the second segment may be formed of a crimped polymer sheet.

For example, the polymer may be prepared with a material selected from the group consisting of polyethylene (PE), polypropylene (PP), polyvinyl chloride (PVC), polyethylene terephthalate (PET), polylactic acid (PLA), cellulose acetate (CA), and aluminum foil.

As the second segment is formed of the woven polymer fiber or the crimped polymer sheet, the second segment may include a single channel or a plurality of channels extending in a longitudinal direction. A channel used herein may refer to a path through which a gas (e.g., air or aerosol) passes.

For example, the second segment formed with the crimped polymer sheet may be formed of a material having a thickness between about 5 µm and about 300 µm, for example, between about 10 µm and about 250 µm. A total surface area of the second segment may be between about 300 square millimeters per millimeter (mm²/mm) and about 1000 mm²/mm. Further, an aerosol cooling element may be formed from a material having a specific surface area between about 10 square millimeters per milligram (mm²/mg) and about 100 mm²/mg.

Meanwhile, the second segment may include a thread containing a volatile flavor ingredient. The volatile flavor ingredient may be menthol, but is not limited thereto. For example, the thread may be filled with an amount of menthol sufficient to provide at least 1.5 milligrams (mg) of menthol to the second segment.

A third segment of the filter rod 22 may be a cellulose acetate filter. A desirable length of the third segment may be adopted from a range of 4 mm to 20 mm. For example, the length of the third segment may be about 12 mm. However, embodiments are not limited thereto.

The third segment may be manufactured such that a flavor is generated by spraying a flavoring liquid onto the third segment in a process of manufacturing the third segment. Alternatively, a separate fiber to which the flavoring liquid is applied may be inserted into the third segment. An aerosol generated in the tobacco rod 21 may be cooled as it passes through the second segment of the filter rod 22, and the cooled aerosol may pass through the third segment into the user. Accordingly, when a flavoring element is added to the third segment, the durability of the flavor to be carried to the user may be enhanced.

In addition, the filter rod 22 may include at least one capsule 23. The capsule 23 may perform a function of generating a flavor or a function of generating an aerosol. For example, the capsule 23 may have a structure in which a liquid containing a fragrance is wrapped with a film. The capsule 23 may have a spherical or cylindrical shape. However, embodiments are not limited thereto.

Referring to FIG. 6, a cigarette 3 may further include a front end plug 33. The front end plug 33 may be disposed on one side of a tobacco rod 31 facing a filter rod 32. The front end plug 33 may prevent the tobacco rod 31 from escaping to the outside, and may also prevent the aerosol liquefied from the tobacco rod 31 during smoking from flowing into an aerosol generating device (e.g., the aerosol generating device 1 of FIGS. 2 to 4).

The filter rod 32 may include a first segment 32a and a second segment 32b. The first segment 32a may correspond to the first segment of the filter rod 22 of FIG. 5, and the second segment 32b may correspond to the third segment of the filter rod 22 of FIG. 5.

A diameter and a total length of the cigarette 3 may correspond to the diameter and the total length of the cigarette 2 of FIG. 5. For example, a length of the front end plug 33 may be about 7 mm, a length of the tobacco rod 31 may be about 15 mm, a length of the first segment 32a may be about 12 mm, and a length of the second segment 32b may be about 14 mm. However, embodiments are not limited thereto.

The cigarette 3 may be wrapped with at least one wrapper 35. The wrapper 35 may have at least one hole through which external air is introduced or internal gas flows out. For example, the front end plug 33 may be wrapped with a first wrapper 35a, the tobacco rod 31 may be wrapped with a second wrapper 35b, the first segment 32a may be wrapped with a third wrapper 35c, and the second segment 32b may be wrapped with a fourth wrapper 35d. In addition, the cigarette 3 may be entirely wrapped again with a fifth wrapper 35e.

In addition, at least one perforation 36 may be formed on the fifth wrapper 35e. For example, the perforation 36 may be formed in an area surrounding the tobacco rod 31. However, embodiments are not limited thereto. The perforation 36 may perform a function of transferring heat generated by the heater 13 shown in FIGS. 3 and 4 to the inside of the tobacco rod 31.

In addition, the second segment 32b may include at least one capsule 34. The capsule 34 may perform a function of generating a flavor or a function of generating an aerosol. For example, the capsule 34 may have a structure in which a liquid containing a fragrance is wrapped with a film. The capsule 34 may have a spherical or cylindrical shape, but is not limited thereto.

The first wrapper 35a may be a combination of general filter wrapping paper and metal foil such as aluminum foil. For example, a total thickness of the first wrapper 35a may be in a range of 45 µm to 55 µm, and desirably, may be 50.3 µm In addition, a thickness of the metal foil of the first wrapper 35a may be in a range of 6 µm to 7 µm, and desirably, may be 6.3 µm. In addition, a basis weight of the first wrapper 35a may be in a range of 50 g/m² to 55 g/m², and desirably, may be 53 g/m².

The second wrapper 35b and the third wrapper 35c may be formed with general filter wrapping paper. For example, the second wrapper 35b and the third wrapper 35c may be porous wrapping paper or non-porous wrapping paper.

For example, the porosity of the second wrapper 35b may be 35000 CU. However, embodiments are not limited thereto. In addition, a thickness of the second wrapper 35b may be in a range of 70 µm to 80 µm, and desirably, may be 78 µm. In addition, a basis weight of the second wrapper 35b may be in a range of 20 g/m² to 25 g/m², and desirably, may be 23.5 g/m².

For example, the porosity of the third wrapper 35c may be 24000 CU. However, embodiments are not limited thereto. In addition, a thickness of the third wrapper 35c may be in a range of 60 µm to 70 µm, and desirably, may be 68 µm. In addition, a basis weight of the third wrapper 35c may be in a range of 20 g/m² to 25 g/m², and desirably, may be 21 g/m².

The fourth wrapper 35d may be formed with polylactic acid (PLA) laminated paper. The PLA laminated paper may refer to three-ply paper including a paper layer, a PLA layer, and a paper layer. For example, a thickness of the fourth wrapper 35d may be in a range of 100 µm to 120 µm, and desirably, may be 110 µm. In addition, a basis weight of the fourth wrapper 35d may be in a range of 80 g/m² to 100 g/m², and desirably, may be 88 g/m².

The fifth wrapper 35e may be formed of sterile paper (e.g., MFW). Here, the sterile paper (MFW) may refer to paper specially prepared to enhance tensile strength, water resistance, smoothness, or the like, compared to general paper. For example, a basis weight of the fifth wrapper 35e may be in a range of 57 g/m² to 63 g/m², and desirably, may be 60 g/m². In addition, a thickness of the fifth wrapper 35e may be in a range of 64 µm to 70 µm, and desirably, may be 67 µm.

The fifth wrapper 35e may have a predetermined material internally added thereto. The predetermined material may be, for example, silicon. However, embodiments are not limited thereto. Silicon may have properties, such as, for example, heat resistance, which is characterized by less change by temperature, oxidation resistance which refers to resistance to oxidation, resistance to various chemicals, water repellency against water, or electrical insulation. However, silicon may not be necessarily used, but any material having such properties described above may be applied (or coated) to the fifth wrapper 35e without limitation.

The front end plug 33 may be formed of cellulose acetate. For example, the front end plug 33 may be manufactured by adding a plasticizer (e.g., triacetin) to cellulose acetate tow. A mono denier of a filament constituting the cellulose acetate tow may be in a range of 1.0 to 10.0, and desirably, may be in a range of 4.0 to 6.0. A mono denier of a filament of the front end plug 33 may be more desirably 5.0. In addition, a cross-section of the filament constituting the front end plug 33 may be Y-shaped. A total denier of the front end plug 33 may be in a range of 20000 to 30000, and desirably, may be in a range of 25000 to 30000. The total denier of the front end plug 33 may be more desirably 28000.

In addition, as needed, the front end plug 33 may include at least one channel, and a cross-sectional shape of the channel may be provided in various ways.

The tobacco rod 31 may correspond to the tobacco rod 21 described above with reference to FIG. 5. Thus, a detailed description of the tobacco rod 31 will be omitted here.

The first segment 32a may be formed of cellulose acetate. For example, the first segment may be a tubular structure including a hollow therein. The first segment 32a may be manufactured by adding a plasticizer (e.g., triacetin) to cellulose acetate tow. For example, a mono denier and a total denier of the first segment 32a may be the same as the mono denier and the total denier of the front end plug 33.

The second segment 32b may be formed of cellulose acetate. A mono denier of a filament constituting the second segment 32b may be in a range of 1.0 to 10.0, and desirably, may be in a range of 8.0 to 10.0. The mono denier of the filament of the second segment 32b may be more desirably 9.0. In addition, a cross-section of the filament of the second segment 32b may be Y-shaped. A total denier of the second segment 32b may be in a range of 20000 to 30000, and desirably, may be 25000.

FIG. 7 is a block diagram illustrating an aerosol generating device according to another example. According to an embodiment, an aerosol generating device 8 may include a housing 80, a sensing unit 81, a solid-type cartridge (or liquid-type cartridge) 82, a coil 83, and a battery 84. The aerosol generating device 8 may operate in a different manner from that of the aerosol generating device 1 described above with reference to FIGS. 2 to 6. For example, the coil 83 may generate heat by receiving energy from the battery 84. The replaceable cartridge 82 may generate an aerosol using the heat generated by the coil 83. A user may suck the generated aerosol through an opening 81 (or a tip portion).

According to an embodiment, the description of the sensing unit 81 may be replaced with the description of the sensing unit 10 provided above with reference to FIGS. 2 to 4.

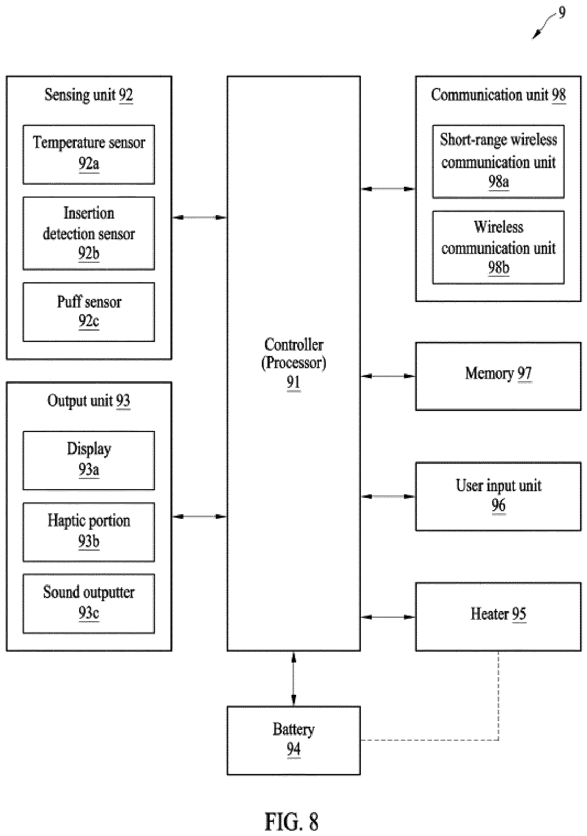

FIG. 8 is a block diagram illustrating an aerosol generating device according to still another example.

According to an embodiment, an aerosol generating device 9 may include a controller 91, a sensing unit 92, an output unit 93, a battery 94, a heater 95, a user input unit 96, a memory 97, and a communication unit 98. However, the internal structure of the aerosol generating device 9 is not limited to what is shown in FIG. 8. It is to be understood by one of ordinary skill in the art to which the disclosure pertains that some of the components shown in FIG. 8 may be omitted or new components may be added according to the design of the aerosol generating device 9.

The sensing unit 92 may sense a status of the aerosol generating device 9 or a status of an environment around the aerosol generating device 9, and transmit sensing information obtained through the sensing to the controller 91. Based on the sensing information, the controller 91 may control the aerosol generating device 9 to control operations of the heater 95, restrict smoking, determine whether an aerosol generating article (e.g., a cigarette, a cartridge, etc.) is inserted, display a notification, and perform other functions.

The sensing unit 92 may include at least one of a temperature sensor 92a, an insertion detection sensor 92b, or a puff sensor 92c. However, embodiments are not limited thereto. For example, the sensing unit 92 may include the sensors of the sensing unit 10 or the sensing unit 81 described above with reference to FIGS. 2 to 8.

The temperature sensor 92a may sense a temperature at which the heater 95 (or an aerosol generating material) is heated. The aerosol generating device 9 may include a separate temperature sensor for sensing a temperature of the heater 95, or the heater 95 itself may perform a function as a temperature sensor. Alternatively, the temperature sensor 92a may be arranged around the battery 94 to monitor a temperature of the battery 94.

The insertion detection sensor 92b may sense whether the aerosol generating article is inserted or removed. The insertion detection sensor 92b may include, for example, at least one of a film sensor, a pressure sensor, a light sensor, a resistive sensor, a capacitive sensor, an inductive sensor, and an infrared sensor, which may sense a signal change by the insertion and/or removal of the aerosol generating article.

The puff sensor 92c may sense a puff from a user based on various physical changes in an airflow path or airflow channel. For example, the puff sensor 92c may sense the puff based on any one of a temperature change, a flow change, a voltage change, and a pressure change.

The sensing unit 92 may further include at least one of a temperature/humidity sensor, an atmospheric pressure sensor, a magnetic sensor, an acceleration sensor, a gyroscope sensor, a position sensor (e.g., a global positioning system (GPS)), a proximity sensor, and a red, green, blue (RGB) sensor (e.g., an illuminance sensor), in addition to the sensors 92a to 92c described above. A function of each sensor may be intuitively inferable from its name by one of ordinary skill in the art, and thus a more detailed description thereof will be omitted here.

The output unit 93 may output information about the status of the aerosol generating device 9 and provide the information to the user. The output unit 93 may include at least one of a display 93a, a haptic portion 93b, and a sound outputter 93c. However, embodiments are not limited thereto. When the display 93a and a touchpad are provided in a layered structure to form a touchscreen, the display 93a may be used as an input device in addition to an output device.

The display 93a may visually provide the information about the aerosol generating device 9 to the user. The information about the aerosol generating device 9 may include, for example, a charging/discharging status of the battery 94 of the aerosol generating device 9, a preheating status of the heater 95, an insertion/removal status of the aerosol generating article, a limited usage status (e.g., an abnormal article detected) of the aerosol generating device 9, or the like, and the display 93a may externally output the information. The display 93a may be, for example, a liquid-crystal display panel (LCD), an organic light-emitting display panel (OLED), or the like. The display 93a may also be in the form of a light-emitting diode (LED) device. The haptic portion 93b may provide the information about the aerosol generating device 9 to the user in a haptic way by converting an electrical signal into a mechanical stimulus or an electrical stimulus. The haptic portion 93b may include, for example, a motor, a piezoelectric element, or an electrical stimulation device.

The sound outputter 93c may provide the information about the aerosol generating device 9 to the user in an auditory way. For example, the sound outputter 93c may convert an electrical signal into a sound signal and externally output the sound signal.

The battery 94 may supply power to be used to operate the aerosol generating device 9. The battery 94 may supply power to heat the heater 95. In addition, the battery 94 may supply power required for operations of the other components (e.g., the sensing unit 92, the output unit 93, the user input unit 96, the memory 97, and the communication unit 98) included in the aerosol generating device 9. The battery 94 may be a rechargeable battery or a disposable battery. The battery 94 may be, for example, a lithium polymer (LiPoly) battery. However, embodiments are not limited thereto.

The heater 95 may receive power from the battery 94 to heat the aerosol generating material. Although not shown in FIG. 8, the aerosol generating device 9 may further include a power conversion circuit (e.g., a direct current (DC)-to-DC (DC/DC) converter) that converts power of the battery 94 and supplies the power to the heater 95. In addition, when the aerosol generating device 9 generates an aerosol in an induction heating manner, the aerosol generating device 9 may further include a DC-to-alternating current (AC) (DC/AC) converter that converts DC power of the battery 94 into AC power.

The controller 91, the sensing unit 92, the output unit 93, the user input unit 96, the memory 97, and the communication unit 98 may receive power from the battery 94 to perform functions. Although not shown in FIG. 8, a power conversion circuit, for example, a low dropout (LDO) circuit or a voltage regulator circuit, which converts power of the battery 94 and supplies the power to respective components, may further be included.

In an embodiment, the heater 95 may be formed of an electrically resistive material that is suitable. The electrically resistive material may be a metal or a metal alloy including, for example, titanium, zirconium, tantalum, platinum, nickel, cobalt, chromium, hafnium, niobium, molybdenum, tungsten, tin, gallium, manganese, iron, copper, stainless steel, nichrome, or the like. However, embodiments are not limited thereto. In addition, the heater 95 may be implemented as a metal heating wire, a metal heating plate on which an electrically conductive track is arranged, a ceramic heating element, or the like. However, embodiments are not limited thereto.

According to another embodiment, the heater 95 may be an induction heater. For example, the heater 95 may include a susceptor that heats the aerosol generating material by generating heat through a magnetic field applied by a coil.

According to an embodiment, the heater 95 may include a plurality of heaters. For example, the heater 95 may include a first heater for heating a cigarette and a second heater for heating a liquid.

The user input unit 96 may receive information input from the user or may output information to the user. For example, the user input unit 96 may include a keypad, a dome switch, a touchpad (e.g., a contact capacitive type, a pressure resistive film type, an infrared sensing type, a surface ultrasonic conduction type, an integral tension measurement type, a piezo effect method, etc.), a jog wheel, a jog switch, or the like. However, embodiments are not limited thereto. In addition, although not shown in FIG. 8, the aerosol generating device 9 may further include a connection interface such as a universal serial bus (USB) interface, and may be connected to another external device through the connection interface such as a USB interface to transmit and receive information or to charge the battery 94.

The memory 97, which is hardware for storing various pieces of data processed in the aerosol generating device 9, may store data processed by the controller 91 and data to be processed by the controller 91. The memory 97 may include at least one type of storage medium of a flash memory type memory, a hard disk type memory, a multimedia card micro type memory, a card type memory (e.g., an SD or XD memory), a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. The memory 97 may store an operating time of the aerosol generating device 9, a maximum number of puffs, a current number of puffs, at least one temperature profile, data associated with a smoking pattern of the user, or the like.

The communication unit 98 may include at least one component for communicating with another electronic device. For example, the communication unit 98 may include a short-range wireless communication unit 98a and a wireless communication unit 98b.

The short-range wireless communication unit 98a may include a Bluetooth communication unit, a Bluetooth low energy (BLE) communication unit, a near field communication unit, a wireless area network (WLAN) (wireless fidelity (Wi-Fi)) communication unit, a ZigBee communication unit, an infrared data association (IrDA) communication unit, a Wi-Fi direct (WFD) communication unit, an ultra-wideband (UWB) communication unit, an Ant+ communication unit, or the like. However, embodiments are not limited thereto.

The wireless communication unit 98b may include a cellular network communication unit, an Internet communication unit, a computer network (e.g., a local area network (LAN) or a wide-area network (WAN)) communication unit, or the like. However, embodiments are not limited thereto. The wireless communication unit 98b may use subscriber information (e.g., international mobile subscriber identity (IMSI)) to identify and authenticate the aerosol generating device 9 in a communication network.

The controller 91 may control the overall operation of the aerosol generating device 9. According to an embodiment, the controller 91 may include at least one processor. The processor may be implemented as an array of a plurality of logic gates, or may be implemented as a combination of a general-purpose microprocessor and a memory in which a program executable by the microprocessor is stored. In addition, it is to be understood by one of ordinary skill in the art to which the present disclosure pertains that it may be implemented in other types of hardware.

The controller 91 may control the temperature of the heater 95 by controlling the supply of power from the battery 94 to the heater 95. For example, the controller 91 may control the supply of power by controlling switching of a switching element between the battery 94 and the heater 95. As another example, a direct heating circuit may control the supply of power to the heater 95 according to a control command from the controller 91.

The controller 91 may analyze a sensing result obtained by the sensing of the sensing unit 92 and control processes to be performed thereafter. For example, the controller 91 may control power to be supplied to the heater 95 to start or end an operation of the heater 95 based on the sensing result obtained by the sensing unit 92. As another example, the controller 91 may control an amount of power to be supplied to the heater 95 and a time for which the power is to be supplied, such that the heater 95 may be heated up to a predetermined temperature or maintained at a desired temperature, based on the sensing result of the sensing unit 92.

The controller 91 may control the output unit 93 based on the sensing result of the sensing unit 92. For example, when the number of puffs counted through the puff sensor 92c reaches a preset number, the controller 91 may inform the user that the aerosol generating device 9 is to be ended soon, through at least one of the display 93a, the haptic portion 93b, and the sound outputter 93c. In an embodiment, the controller 91 may control a power supply time and/or a power supply amount for the heater 95 according to a status of the aerosol generating article sensed by the sensing unit 92. For example, when an aerosol generating substrate is in an over-humidified status, the controller 91 may control the power supply time for an inductive coil to increase a preheating time, compared to a case where the aerosol generating substrate is in a general status. FIG. 9 is a diagram illustrating a configuration of a user terminal according to an embodiment. A user terminal 900 includes a communication unit 910, a processor 920, and a memory 930. For example, the user terminal 900 may be the user terminal 110 described above with reference to FIG. 1.

The communication unit 910 may be connected to the processor 920 and the memory 930 and transmit and receive data to and from the processor 920 and the memory 930. The communication unit 910 may be connected to another external device and transmit and receive data to and from the external device. Hereinafter, transmitting and receiving "A" may refer to transmitting and receiving "information or data indicating A".

The communication unit 910 may be implemented as circuitry in the user terminal 900. For example, the communication unit 910 may include an internal bus and an external bus. As another example, the communication unit 910 may be an element configured to connect the user terminal 900 to an external device. The communication unit 910 may be an interface. The communication unit 910 may receive data from the external device and transmit the data to the processor 920 and the memory 930.

The processor 920 may process the data received by the communication unit 910 and data stored in the memory 930. A "processor" may be a hardware-implemented data processing device having a physically structured circuit to execute desired operations. The desired operations may include, for example, code or instructions included in a program. The hardware-implemented data processing device may include, for example, a microprocessor, a central processing unit (CPU), a processor core, a multi-core processor, a multiprocessor, an application-specific integrated circuit (ASIC), and a field-programmable gate array (FPGA).

The processor 920 may execute computer-readable code (e.g., software) stored in a memory (e.g., the memory 930) and instructions triggered by the processor 920.

The memory 930 may store the data received by the communication unit 910 and the data processed by the processor 920. For example, the memory 930 may store the program (or an application or software). The program to be stored may be a set of syntaxes that are coded and executable by the processor 920 to manage smoking information of a user.

According to an aspect, the memory 930 may include at least one volatile memory, non-volatile memory, RAM, flash memory, a hard disk drive, and an optical disc drive.

The memory 930 may store an instruction set (e.g., software) for operating the user terminal 900. The instruction set for operating the user terminal 900 may be executed by the processor 920. The communication unit 910, the processor 920, and the memory 930 will be described in detail below with reference to FIGS. 10 to 12.

FIG. 10 is a flowchart illustrating a method of outputting a status of a suction ability performed by a user terminal according to an embodiment.

Operations 1010 to 1050 described below may be performed by the user terminal 900 described above with reference to FIG. 9.

In operation 1010, the user terminal 900 may receive, from the additional device, inhalation information generated by a sensor of an additional device. The additional device may be an electronic cigarette (e.g., an aerosol generating device). The electronic cigarette may be the aerosol generating device 120 of FIG. 1, the aerosol generating device 1 of FIGS. 2 to 4, the aerosol generating device 8 of FIG. 7, or the aerosol generating device 9 of FIG. 8. In terms of the relationship with a user terminal (e.g., the user terminal 110 of FIG. 1 or the user terminal 900 of FIG. 9), the electronic cigarette may be referred to as an additional device.

According to an embodiment, the additional device may detect inhalation by a user using one or more sensors of the additional device and generate inhalation information on the inhalation by the user. For example, the inhalation information may include at least one of an average flow rate of airflow according to the inhalation by the user, a cross-sectional area of a channel through which airflow passes, or a duration of resistance to draw.

Before operation 1010 is performed, the user terminal 900 and the additional device may establish a link using short-range wireless communication (e.g., Bluetooth), and the user terminal 900 may receive the inhalation information through the generated link.

In operation 1020, the user terminal 900 may determine a target suction ability of the user based on the inhalation information. For example, the suction ability may include at least one of a suction intensity and a suction time. A target suction intensity and a target suction time may be determined as the target suction ability based on the average flow rate of airflow, the cross-sectional area of the channel through which airflow passes, and the duration of resistance to draw included in the inhalation information.

In operation 1030, the user terminal 900 may determine a status of the user by evaluating the suction ability based on a preset reference suction ability. For example, the reference suction ability may be a suction ability that is appropriate for the user.

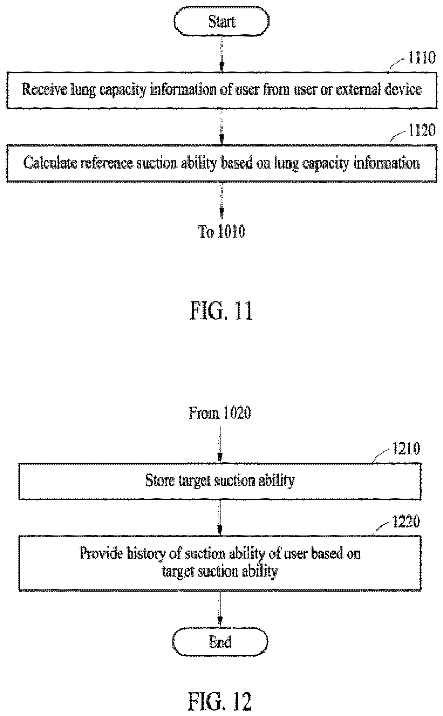

According to an embodiment, the reference suction ability may be preset, and a method of presetting the reference suction ability is described in detail below with reference to FIG. 11. For example, the status of the user may indicate a change in the suction intensity. Whether the target suction intensity of the user is weaker or stronger than the reference suction intensity may be determined to be the status of the user.

For example, the status of the user may indicate a change in the suction time. Whether the target suction time is shorter or longer than the reference suction time may be determined to be the status of the user.

In operation 1040, the user terminal 900 may generate a notification message based on the status of the user. For example, the content of the notification message may be "Please increase the suction intensity", "Please decrease the suction intensity slightly", "Please suck for about one more second", or "Please shorten the suction time slightly".

In operation 1050, the user terminal 900 may output the generated notification message. For example, the notification message may be output through a speaker. As another example, the notification message may be output through a display.

The user terminal 900 may determine a current status of the suction ability of the user and provide guidance to the user to achieve an appropriate suction ability, and the user may enhance the sensation of smoking and increase smoking satisfaction by following the guidance.

FIG. 11 is a flowchart illustrating a method of calculating a reference suction ability according to an example.

According to an embodiment, operations 1110 and 1120 described below may be performed by the user terminal 900 before operation 1010 described above with reference to FIG. 10 is performed.

In operation 1110, the user terminal 900 may receive lung capacity information of a user from the user or an external device. For example, the lung capacity information may include a maximum amount of inhalation, a maximum amount of exhalation, a remaining amount, and an amount of forced exhalation.

For example, the user terminal 900 may receive the lung capacity information from the user through a user interface. As another example, the user terminal 900 may receive the lung capacity information from an external server (e.g., a medical data management server) linked with the user terminal 900. As still another example, the user terminal 900 may instruct the user to measure his/her lung capacity while the user is not smoking using the additional device (e.g., using the additional device with an empty tube filter), and the additional device may transmit the measured lung capacity information of the user to the user terminal 900.

In operation 1120, the user terminal 900 may calculate a reference suction ability based on the lung capacity information. The reference suction ability may be a suction ability of the user while the user is not smoking. Additionally, the user terminal 900 may calculate the reference suction ability further based on demographic information such as the age and gender of the user in addition to the lung capacity information. The reference suction ability may include parameters personalized to the user. For example, the parameters may include an appropriate suction intensity and an appropriate suction time.

According to an embodiment, the parameters of the reference suction ability may be calculated differently depending on a type of a heater used by the additional device, a characteristic of a generated aerosol, or a type of a cigarette. It may be possible to provide the user with a high level of smoking satisfaction by adjusting the parameters of the reference suction ability according to a smoking environment.

FIG. 12 is a flowchart illustrating a method of providing a history of a suction ability of a user according to an example.

According to an embodiment, operations 1210 and 1220 below may be further performed by the user terminal 900 after operation 1020 described above with reference to FIG. 10 is performed. Operations 1210 and 1220 may be performed independently or in parallel with operations 1030 to 1050 of FIG. 10.

In operation 1210, the user terminal 900 may store a determined current target suction ability. According to an embodiment, the user terminal 900 may receive, from an additional device, not only a target suction ability but also pieces of biometric information measured by biosensors (e.g., a blood pressure sensor, an electrocardiogram sensor, a blood oxygen saturation sensor, etc.) of the additional device, and may store the pieces of biometric information.

In operation 1220, the user terminal 900 may provide a history of a suction ability of the user based on the target suction ability. For example, the user terminal 900 may output a graph depicting a trend of a suction ability that changes over time. The user may monitor his/her health status through the changing trend of the suction ability.

According to an embodiment, the user terminal 900 may provide the user with an additional notification when the determined current target suction ability is less than or equal to a preset threshold suction ability.

According to an embodiment, the user terminal 900 may provide the user with a history of the pieces of biometric information together with the history of the suction ability. The user may continuously monitor his/her health status through various pieces of information.

The methods according to the embodiments may be recorded in computer-readable media including program instructions to implement various operations of the embodiments. The computer-readable media may include, alone or in combination with the program instructions, data files, data structures, and the like. The program instructions recorded on the media may be those specially designed and constructed for the purposes of embodiments, or they may be of the kind well-known and available to one of ordinary skill in the computer software arts. Examples of computer-readable media include magnetic media such as hard disks, floppy disks, and magnetic tape; optical media such as CD-ROM or DVDs; magneto-optical media such as floptical disks; and hardware devices that are specially configured to store and perform program instructions, such as ROM, RAM, flash memory, and the like. Examples of program instructions include both machine code, such as produced by a compiler, and files containing higher-level code that may be executed by the computer using an interpreter. The above-described hardware devices may be configured to act as one or more software modules in order to perform the operations of the embodiments, or vice versa.

The software may include a computer program, a piece of code, an instruction, or one or more combinations thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software may also be distributed over network-coupled computer systems so that the software may be stored and executed in a distributed fashion. The software and data may be stored by one or more computer-readable recording mediums.

Although the embodiments have been described with reference to the limited drawings, one of ordinary skill in the art may apply various technical modifications and variations based thereon. For example, suitable results may be achieved if the described techniques are performed in a different order and/or if components in a described system, architecture, device, or circuit are combined in a different manner or replaced or supplemented by other components or their equivalents.

Therefore, other implementations, other embodiments, and equivalents to the claims are also within the scope of the following claims.

## Claims

1. A method of outputting a status of a suction ability, performed by an electronic device, the method comprising:
receiving, from an additional device connected to the electronic device, inhalation information generated based on one or more sensors in the additional device;
determining a target suction ability of a user based on the inhalation information;
determining a status of the user by evaluating the target suction ability based on a reference suction ability preset in the electronic device;
generating a notification message based on the status; and
outputting the notification message.

2. The method of claim 1, wherein the inhalation information comprises at least one of an average flow rate of airflow according to inhalation by the user, a cross-sectional area of a channel through which airflow passes, and a duration of resistance to draw.

3. The method of claim 1, further comprising:
calculating the reference suction ability based on lung capacity information of the user received from the user or an external device.

4. The method of claim 1, wherein the determining of the status of the user by evaluating the target suction ability based on the reference suction ability preset in the electronic device comprises determining the status of the user by comparing a reference suction intensity and a reference suction time of the reference suction ability with a target suction intensity and a target suction time of the target suction ability.

5. The method of claim 1, wherein the notification message indicates a message to adjust at least one of a suction intensity and a suction time.

6. The method of claim 1, further comprising:
storing the target suction ability; and
providing the user with a history of a suction ability of the user based on the target suction ability.

7. The method of claim 1, wherein
the electronic device is a mobile communication terminal, and
the additional device is an electronic cigarette.

8. A computer-readable storage medium storing a program for executing the method of claim 1.

9. An electronic device comprising:
a memory configured to store a program for outputting a status of a suction ability; and
a processor configured to perform the program,
wherein the processor is configured to:
receive, from an additional device connected to the electronic device, inhalation information generated based on one or more sensors in the additional device,
determine a target suction ability of a user based on the inhalation information;
determine a status of the user by evaluating the target suction ability based on a reference suction ability preset in the electronic device;
generate a notification message based on the status; and
output the notification message.

10. The electronic device of claim 9, wherein
the electronic device is a mobile communication terminal, and
the additional device is an electronic cigarette.
